Europäisches Patentamt

European Patent Office

Office européen des brevets

(11) Publication number: **0 361 797 B1**

# EUROPEAN PATENT SPECIFICATION

(45) Date of publication of patent specification: **30.08.95**

(51) Int. Cl.[6]: **C03C 10/00**, C03C 14/00, C04B 35/26, A61N 1/40

(21) Application number: **89309645.3**

(22) Date of filing: **21.09.89**

(54) **Heat-generating ceramics body for hyperthermia and method of producing the same.**

(30) Priority: **26.09.88 JP 238786/88**

(43) Date of publication of application:
**04.04.90 Bulletin 90/14**

(45) Publication of the grant of the patent:
**30.08.95 Bulletin 95/35**

(84) Designated Contracting States:
**DE FR GB IT NL**

(56) References cited:
**EP-A- 0 040 512
FR-A- 2 243 915
US-A- 4 043 821**

**INTERNATIONAL CONGRESS ON GLASS,
Kyoto, 8th December 1974, part 2, pages 9-30
-9-41, Ceramic Society of Japan, Kyoto, JP;
L.L. HENCH: "Biomedical applications and
glass corrosion"**

(73) Proprietor: **KYOTO UNIVERSITY
36 Yoshidahon-Machi
Sakyo-ku
Kyoto City
Kyoto-Fu (JP)**

(72) Inventor: **Kokubo, Tadashi
46-1, Yokoyama
Shimokaiinji
Nagaokakyo City
Kyoto-Fu (JP)**
Inventor: **Yamamuro, Takao
100-33, Kitanokuchi
Mozume-Cho
Muko City
Kyoto-Fu (JP)**
Inventor: **Ohura, Koichiro 603, Takehana
Heights
6, Takehanajizoji-Minami-Machi
Yamashina-Ku
Kyoto-City
Kyoto-Fu (JP)**
Inventor: **Ebisawa, Yukihiro
19-10, Tomio-Izumigaoka
Nara City
Nara Pref. (JP)**

(74) Representative: **Cresswell, Thomas Anthony et al**
**J.A. KEMP & CO.**
**14 South Square**
**Gray's Inn**
**London WC1R 5LX (GB)**

**Description**

The present invention relates to a heat-generating ceramics body, particularly a heat-generating ceramics body for hyperthermia of cancers, and methods of producing the same.

Generally, cancer cells are susceptible to heat and die if heated to around 43°C. Because a cancer affected tissue allows a smaller flow of blood than the surrounding tissue, the cancer affected tissue can be heated more easily that the surrounding tissue. Therefore, hyperthermia which selectively and locally heats a cancer affected tissue is a very effective means for curing cancer.

Heretofore, as methods of selectively and locally heating cancer-affected tissues, heating with, for example, hot water, infrared rays, ultrasonic waves, microwaves and high frequency waves have been attempted (JP-A-58-209,530 and JP-A-61-158,931). However, all these methods are not effective for deep cancers, such as bone tumours.

For example, a method is known comprising pressing the surface of a living body near a cancer site between a couple of electrode bags each containing an electrode in saline, and applying a high frequency electric current of about 8 MHz directly through the living body across the electrode bags to heat the cancer cells. However, the hyperthermia apparatus for carrying out such a method can heat only those cancer cells within about 15 cm from the body surface. Moreover, not only cancer cells, but also normal cells are heated, so that normal cells are adversely influenced by the hyperthermia if it is used continuously or for a long time.

Therefore, in order to heat cancer cells solely, a method has been attempted comprising embedding a ferromagnetic body, such as a metal rod, needle or powder in an affected part of a living body, and generating heat therefrom in an alternating current magnetic field. However, metallic substances have high electrical conductivities and are easily dissolved in fluids of living bodies to liberate noxious metallic ions, so that they have low heat-generating efficiencies and are not suited to being embedded in living bodies.

EP-A-0 040 512 discloses a ceramic suitable for localised heating and treatment of cancers, which ceramic has embedded therein magnetic iron-containing crystals.

The present invention seeks to provide a ceramics body to be embedded in living tissues, which is innocuous and stable for a long period when embedded in living tissues and exhibits a splendid affinity to human tissues therearound. The body can be used in hyperthermia treatment to selectively and efficiently heat cancer-affected tissues including deep tissues of living bodies.

The inventors have found that materials such as hydroxyapatite and glass or crystallized glass consisting of CaO and $SiO_2$ provide or form a hydroxy-apatite layer resembling bone on the surface of a ceramic body in living tissues, which layer exhibits a splendid affinity to living tissues such that it bonds naturally to bone therearound. These materials incorporate phosphoric ions from fluids of living bodies to form a layer of hydroxyapatite ($Ca_{10}(PO_4)_6(OH)_2$) on the surface thereof, even though they do not possess phosphoric oxide therein. The hydroxyapatite layer has such good affinity to living bodies that it can chemically bond directly to bone tissue in living bodies, and exhibits a superior affinity to soft tissues, such as muscle and skin. Prior heat-generating bodies in living bodies are considered as foreign materials by the living bodies and become encapsulated with fibrous tissues, even if they do not release noxious metallic ions when dissolved in living body fluids.

The present invention accordingly provides a ceramics body for use in hyperthermia treatment, which body consists of 20-90 wt % of ferromagnetic ferrite particles (A), 80-10 wt % of an inorganic material (B) as a matrix in which the particles are dispersed, and optionally up to 10 wt % of another component (C) which is $Li_2O$, $K_2O$, MgO, SrO, $B_2O_3$, $Al_2O_3$, $TiO_2$, $ZrO_2$, $Nb_2O_5$, $Ta_2O_5$ or $CaF_2$, the inorganic material (B) consisting of at least one of wollastonite, dicalciumsilicate, hydroxyapatite, a glass consisting of CaO and $SiO_2$, and a crystallized glass consisting of CaO and $SiO_2$, such that hydroxyapatite is either present in a surface layer or is formed in a surface layer when the body is embedded in living tissue.

The hydroxyapatite surface layer is referred to below as a bioactive inorganic layer. When the ceramics body of the invention is embedded in living tissues of the human body it is not perceived as a foreign material by the surrounding tissues, and the ferrite particles are highly efficient in providing magnetic induction heat generation in an alternating magnetic field because heat loss occurs through magnetic hysteresis only.

The ceramics body of the invention can be produced by at least the following two ways (1) and (2). (1) A method which comprises sintering a powder mixture composition consisting of 20-90 wt % of ferromagnetic ferrite particles (A), 80-10 wt % of an inorganic material (B) of glass or crystallized glass, and optionally up to 10 wt % of the other component (C).

The ferromagnetic ferrite particles are preferably magnetite ($Fe_3O_4$), lithiumferrite ($LiFe_5O_8$) or magnesiumferrite ($MgFe_2O_4$). Other ferromagnetic ferrites can be used, so long as they do not liberate noxious ions in living bodies.

Ferromagnetic ferrite is an essential component for generating heat in an alternating current magnetic field and it is essential to use an amount of at least 20 wt % to obtain a practical level of heating. If the ferromagnetic ferrite is used in the composition in an amount of more than 90 wt %, the ferrite particles are not perfectly coated with the bioactive material, and therefore the amount of ferromagnetic ferrite is limited to not over 90 wt %.

The bioactive component is an essential component of the ceramics body for exhibiting a good affinity to human tissues in a living body, and particularly for exhibiting bonding to bone if the ceramics body is embedded in a living body near bone. In order to completely coat the ferromagnetic particles with the inorganic material, the material should be used in an amount of at least 10 wt % in the composition. However, if the amount of the inorganic material exceeds 80 wt %, an insufficient amount of heat is generated, and so the amount of inorganic material is limited to not over 80 wt %.

For sintering the composition, any temperature can be used so long as the composition is sintered. (2) A method of producing a ceramics body which comprises cooling a melt of a composition consisting of 10-60 wt % of CaO, 5-50 wt % of $SiO_2$, 10-80 wt % of $Fe_2O_3$, and optionally up to 10 wt % of the other component (C), to precipitate ferrite particles (A) during the cooling, or to form a glass, and then optionally reheating the composition to precipitate ferrite particles.

In this method, if the amount of CaO is less than 10 wt %, the glass or crystallized product containing ferrite particles does not exhibit a good affinity to living bodies. If the amount of CaO exceeds 60 wt %, the melting point of the composition rises so much that a melt of a uniform composition is hard to produce. Hence, the amount of CaO is 10-60 wt %.

If the amount of $SiO_2$ is less than 5 wt %, a melt of a uniform composition can not be obtained. If the amount of $SiO_2$ exceeds 50 wt %, the content of $Fe_2O_3$ is relatively decreased in the composition, and so the amount of precipitated ferrite particles becomes small causing poor heat generation. Thus, the amount of $SiO_2$ is 5-50 wt %.

If the amount of $Fe_2O_3$ is less than 10 wt %, only a small amount of ferrite crystals can be precipitated from the melt, so that good heat-generation can not be obtained. If the amount of $Fe_2O_3$ exceeds 80 wt %, the bioactive glass or crystallized glass consisting of CaO and $SiO_2$ and having a good affinity to living bodies can not completely cover the ferromagnetic ferrite particles. Therefore, the amount of $Fe_2O_3$ is 10-80 wt %. In order to precipitate a ferrite other than magnetite, a metal oxide which forms the other ferrite may be included in the composition in addition to $Fe_2O_3$.

The composition of the ceramics body can form a coating of hydroxyapatite on its surface, even though the composition does not contain $P_2O_5$.

The composition optionally contains up to 10 wt % of $Li_2O$, $K_2O$, MgO, SrO, $B_2O_3$, $Aℓ_2O_3$, $TiO_2$, $ZrO_2$, $Nb_2O_5$, $Ta_2O_5$ or $CaF_2$. If the amount of these components exceeds 10 wt % of the composition, the relative amount of $Fe_2O_3$ in the composition decreases and the content of precipitated ferrite particles in the product is decreased, and so heat-generation is decreased. Further, the contents of CaO and $SiO_2$ are decreased, and so the affinity to living bodies is decreased. Therefore, the amount of the optional components is limited to a sum of up to 10 wt %. Thus, the sum of CaO, $SiO_2$ and $Fe_2O_3$ is at least 90 wt %.

If the melt is cooled at a relatively small cooling rate, ferromagnetic ferrite particles can be precipitated from the melt during cooling. By selecting the cooling rate appropriately, the amount and the diameter of the ferrite particles can be controlled appropriately. By controlling the amount and the diameter of the ferrite particles appropriately, the heat-generating property of the composition can be controlled appropriately. If the melt is rapidly cooled or quenched, a glass can be obtained, and the glass can be reheated to precipitate ferrite particles. The temperature in the heating at which the ferromagnetic ferrite particles precipitate can be determined by measuring preliminarily the exothermic peak in a differential thermal analysis of the glass. By selecting heating conditions appropriately, the amount and the diameter of the ferrite particles to be precipitated can be controlled appropriately, so that the heat-generating property of the composition can be controlled appropriately.

When the melt is cooled to precipitate ferrite, or in the case when the glass obtained from the melt is heated to precipitate ferrite, crystals other than ferrite, such as wollastonite (CaO•$SiO_2$), dicalcium silicate (2CaO•$SiO_2$), or hydroxyapatite ($Ca_{10}(PO_4)_6(OH)_2$), may be precipitated without hindrance. The phase coating and surrounding the ferrite particles varies depending on the cooling conditions of the melt and the heating conditions of the glass.

As explained above, in the heat-generating ceramics body of the present invention, the ferromagnetic ferrite particles are coated with a bioactive inorganic material layer which exhibits a splendid affinity to surrounding tissues in living bodies and remains safely for a long period of time.

4

The heat-generating ceramics body, which can be induction heated, can be used in a fine powder form, a shaped bulk form, or a fibrous form and can be transferred to cancer affected tissues of living bodies by means of intravenous injection, subcutaneous injection, oral administration, or surgical embedment depending on the form thereof, and then placed together with the cancer affected tissue in an alternating magnetic field. The ferromagnetic ferrite particles generate heat with magnetic hysteresis loss to heat and cure the cancer affected tissue.

For a better understanding of the present invention, reference is made to the accompanying drawings, in which:

Fig. 1 is a schematic view of the heat-generating ceramics body of the present invention;

Fig. 2 is a graph of a magnetization curve showing the principle of heat generation by the heat-generating ceramics body of the present invention;

Fig. 3 is a graph of heat generation curves of examples of the heat-generating ceramics body of the present invention;

Fig.4 is a schematic view of the disposition of the heat-generating ceramics body at the time of temperature elevation; and

Fig. 5 is a graph showing characteristic results thereof.

Hereinafter, the present invention will be explained in more detail with reference to Examples.

Examples 1-15

Examples of the method of producing the heat-generating ceramics body for hyperthermia of the present invention are shown in Table 1 and 2. Table 1 shows Examples corresponding to claim 3 and Table 2 to claim 5, respectively. In Table 1, the mixing ratio of the bioactive inorganic material and the ferrite particles is selected as 1:1 for convenience, but the present invention is not limited thereto.

In Examples 1-3 of Table 1, the hydroxyapatite is produced by reaction of slaked lime and phosphoric acid in an aqueous solution. Ferrite particles are synthesized by a wet process. When the ferrite particles are of $Fe_3O_4$, the $Fe_3O_4$ is produced in the following way. Ferrous sulfate is an aqueous solution of sodium hydroxide at $80°C$ is reacted with an oxygen stream. After 1 day, the reaction is stopped, and the reacted ferrous sulfate or $Fe_3O_4$ is rinsed well with water, then dried, and kneaded with stearic acid. Hydroxyapatite and the fine powdery $Fe_3O_4$ is mixed homogeneously in a mixer mill, shaped under a pressure of 400 $kg/cm^2$, and sintered in an $N_2$ stream. When the ferrite particles are $LiFe_5O_8$ or $MgFe_2O_4$, the shaped bodies can be sintered in air.

Table 1

| Example | Bioactive inorganic layer | Ferrite | Coercive force (Oe) | Magnetization (emu/g) |
|---------|---------------------------|---------|---------------------|------------------------|
| 1 | hydroxyapatite | $Fe_3O_4$ | 100 | 40 |
| 2 | hydroxyapatite | $LiFe_5O_8$ | 80 | 10 |
| 3 | hydroxyapatite | $MgFe_2O_4$ | 100 | 30 |

Batches corresponding to the compositions of Examples 4 and 5 of Table 2 are prepared by putting in platinum crucibles the raw materials of an oxide, a carbonate, a hydroxide or a fluoride, and melting the materials in an electric furnace at 1,300-1,550°C for 2 hrs. The melts are casted in molds of a depth of 5 mm placed on an electric heater, and cooled at a controlled cooling rate by adjusting an electric current supplied to the heater to precipitate ferrite particles during cooling of the melts. In this way, relatively large ferrite crystals of a diameter of about 1 $\mu$m can be more easily obtained than in the case of vitrifying once and then crystallizing which will be described later.

5

Table 2

| Example | Composition (wt %) | | | | | Magnetic property | |
|---|---|---|---|---|---|---|---|
| | CaO | SiO$_2$ | Fe$_2$O$_3$ | Rest | | Coercive force (Oe) | Magnetization (emu/g) |
| 4 | 26.2 | 7.6 | 66.2 | --- | | 50 | 43 |
| 5 | 24.1 | 25.9 | 50.0 | --- | | 100 | 32 |
| 6 | 24.2 | 25.8 | 50.0 | --- | | 440 | 35 |
| 7 | 19.3 | 20.7 | 60.0 | --- | | 110 | 41 |
| 8 | 29.0 | 31.0 | 37.0 | Li$_2$O | 3.0 | 390 | 26 |
| 9 | 24.1 | 25.9 | 47.0 | MgO | 3.0 | 350 | 27 |
| 10 | 26.5 | 28.5 | 40.0 | SrO | 5.0 | 400 | 30 |
| 11 | 21.6 | 23.4 | 50.0 | B$_2$O$_3$ | 5.0 | 430 | 29 |
| 12 | 24.1 | 25.9 | 45.0 | Aℓ$_2$O$_3$ | 5.0 | 300 | 27 |
| 13 | 21.6 | 23.4 | 50.0 | TiO$_2$ | 5.0 | 450 | 31 |
| 14 | 16.5 | 18.5 | 60.0 | Ta$_2$O$_5$ | 5.0 | 150 | 36 |
| 15 | 28.0 | 30.0 | 40.0 | CaF$_2$ | 2.0 | 440 | 30 |

Batches corresponding to the compositions of Examples 6-15 of Table 2 are prepared by putting in platinum crucibles raw materials of an oxide, a carbonate, a hydroxide, or a fluoride, and melting the materials in an electric furnace at 1,300-1,550°C for 2 hrs. Then, the melts are cast on iron plates or sandwiched and cooled between a pair of rotating rollers to produce glasses, and then pulverized into powders of a grain or particle size of -325 mesh (sieve opening 44 μm). These powders are shaped under pressure into desired forms, heated in an electric furnace from room temperature up to a ferrite-precipitating temperature (about 1,000°C) at a temperature raising rate of 5°C/min to precipitate ferrite at the chosen precipitation temperature, and then left to cool in the electric furnace to which the electric current is switched off. The same results can be obtained when the glasses are not pulverized and heat treated in plate shapes.

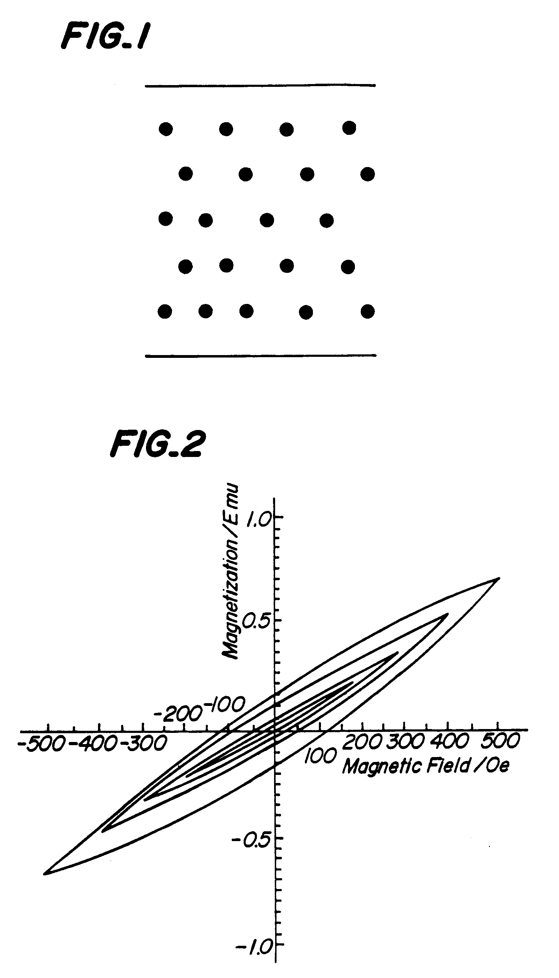

All the heat-generating ceramics bodies obtained by the above methods have a structure wherein the ferromagnetic ferrite particles are embedded in a bioactive inorganic material, as shown in Fig. 1. In Fig. 1, the symbol ● represents a ferromagnetic ferrite particle and the area surrounding the symbol ● represents a bioactive inorganic solid material.

The thus obtained heat-generating ceramics bodies were pulverized, and their magnetization characteristics measured under various magnetic fields and ±100-±500 Oe using a small vibration type magnetization meter (VSM-3 type produced by TOEI). The magnetization characteristics of the heat-generating body of the composition of Example 6 of Table 2 is shown in Fig. 2. The same measurements were effected on the heat-generating body of the composition of Example 7. Based on these results, the amount P of heat generated in a magnetic field of 100 kHz of various strengths was calculated from the equation:

$$P(w/g) = [f \cdot \int HdB] \times 10^{-7}$$

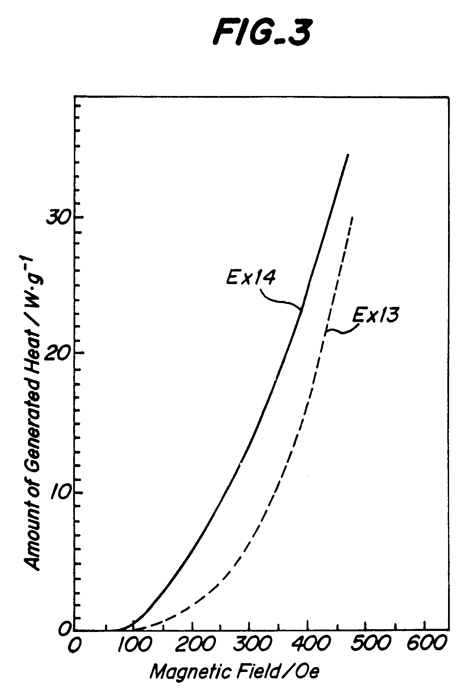

to obtain the results shown in Fig. 3. In the equation, f is the frequency (Hz), H is the strength of the magnetic field and B is the magnitude (emu/g) of magnetization. As seen from Fig. 3, the heat-generating body of Example 7 exhibits a larger heat generation in a low magnetic field that the heat-generating body of Example 6. This is because the heat-generating body of Example 7 exhibits a lower coercive force and a higher saturated magnetization than that of the latter.

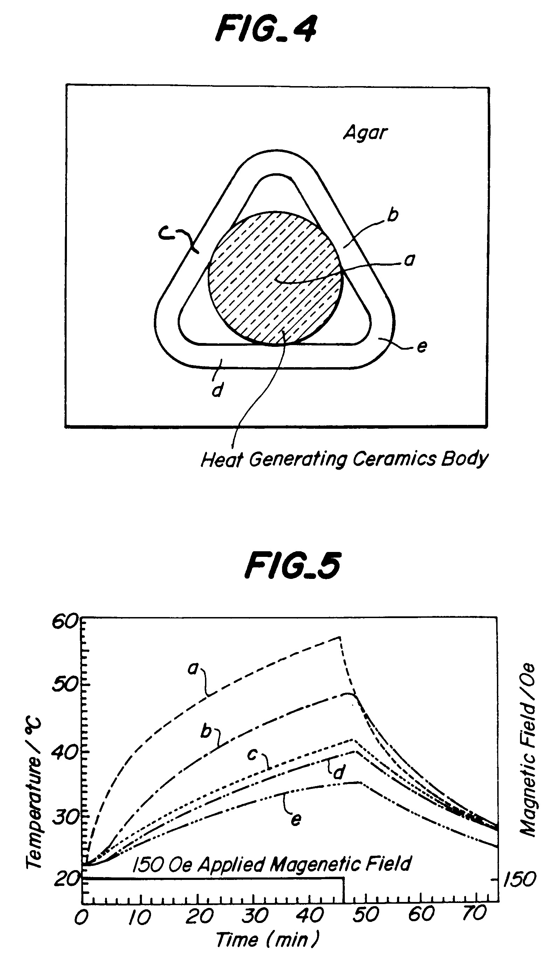

2 g of the particles of the heat-generating ceramics body of Example 6 of Table 2 of a particle size of -350 mesh (44 μm) were embedded in the central portion of a piece of agar of a size of 40 mmΦx110 mm, and the agar was placed in the central portion of an air-core coil of a length of 240 mm. An electric current of a maximum of 5 kW was passed through the coil in a magnetic field of 100 kHz, and the temperature change of the heat-generating body was measured by a copper-constantan thermocouple inserted into the

6

central part of the coil. Agar has a specific heat and density similar to those of tissues of living bodies. By this experiment, it became apparent that the temperature of the central portion of the heat-generating body in a magnetic field of 170 Oe reaches 43°C within about 5 min from the initiation of the application of the electric current. Next, 3 g of the above heat-generating body was embedded in the central portion of a vertebra of a dead pig as shown in Fig. 4, and thermocouples were arranged on the central part and the circumferential parts thereof at the locations a, b, c, d, and e as shown in Fig. 4, and the whole lot was embedded in agar. The agar was placed in an air-core coil in the same manner as described above, and an electric current of a maximum of 5 kW was passed therethrough in a magnetic field of 100 kHz. The temperature changes at the respective locations of the thermocouples were measured. Fig. 5 shows the temperature changes at the respective locations a, b, c, d, and e of Fig. 4 when an electric current of 3 kW was passed in a magnetic field of 150 Oe. As seen from Fig. 5, bone can be heated to a temperature of 41-44°C which is necessary to annihilate cancer cells, in the manner described in Fig. 4.

As apparent from the foregoing detailed explanations, the induction heated ceramics body for hyperthermia of the present invention exhibits superior heat-generation and good affinity to living bodies. The ceramics body is particularly effective for deep cancers, such as bone tumors, and does not liberate noxious ions, such as metallic ions, when dissolved in fluids of living bodies. Thus, the ceramics body is remarkably effective in curing cancer affected tissues over a long period or continuously.

## Claims

1. A ceramics body for use in hyperthermia treatment, which body consists of 20-90 wt % of ferromagnetic ferrite particles (A), 80-10 wt % of an inorganic material (B) as a matrix in which the particles are dispersed, and optionally up to 10 wt % of another component (C) which is $Li_2O$, $K_2O$, MgO, SrO, $B_2O_3$, $Al_2O_3$, $TiO_2$, $ZrO_2$, $Nb_2O_5$, $Ta_2O_5$ or $CaF_2$, the inorganic material (B) consisting of at least one of wollastonite, dicalciumsilicate, hydroxyapatite, a glass consisting of CaO and $SiO_2$, and a crystallized glass consisting of CaO and $SiO_2$, such that hydroxyapatite is either present in a surface layer or is formed in a surface layer when the body is embedded in living tissue.

2. A ceramics body according to claim 1 wherein the ferrite particles are of magnetite ($Fe_3O_4$), lithium ferrite ($LiFe_5O_8$) or magnesium ferrite ($MgFe_2O_4$).

3. A method of producing a ceramics body as claimed in claim 1 which comprises sintering a powder mixture composition consisting of 20-90 wt % of ferromagnetic ferrite particles (A), 80-10 wt % of an inorganic material (B) of glass or crystallized glass, and optionally up to 10 wt % of the other component (C).

4. A method according to claim 3 wherein the ferromagnetic ferrite particles are of magnetite ($Fe_3O_4$)-,lithium ferrite ($LiFe_5O_8$) or magnesium ferrite ($MgFe_2O_4$).

5. A method of producing a ceramics body as claimed in claim 1 which comprises cooling a melt of a composition consisting of 10-60 wt % of CaO, 5-50 wt % of $SiO_2$, 10-80 wt % of $Fe_2O_3$, and optionally up to 10 wt % of the other component (C), to precipitate ferrite particles (A) during the cooling, or to form a glass, and then optionally reheating the composition to precipitate ferrite particles.

## Patentansprüche

1. Keramischer Körper zur hyperthermischen Behandlung, dadurch gekennzeichnet, daß er besteht aus 20 bis 90 Gew.-% ferromagnetischen Ferritteilchen (A), 80 bis 10 Gew.-% eines anorganischen Materials (B) als Matrix, der die Teilchen dispergiert sind, und gegebenenfalls bis zu 10 Gew.-% einer anderen Komponente (C), die $Li_2O$, $K_2O$, MgO, SrO, $B_2O_3$, $Al_2O_3$, $TiO_2$, $ZrO_2$, $Nb_2O_5$, $Ta_2O_5$ oder $CaF_2$ ist, und worin das anorganische Material (B) aus mindestens einer der Komponenten Wollastonit, Dicalciumsilikat, Hydroxyapatit, einem aus CaO und $SiO_2$ bestehenden Glas, und einem aus CaO und $SiO_2$ bestehenden kristallisierten Glas besteht, so daß Hydroxyapatit entweder in einer Oberflächenschicht vorhanden ist oder in einer Oberflachenschicht gebildet wird, wenn der Körper in lebendes Gewebe eingebettet wird.

2. Keramischer Körper nach Anspruch 1, dadurch gekennzeichnet, daß die Ferritteilchen Magnetit ($Fe_3O_4$),Lithiumferrit ($LiFe_5O_8$) oder Magnesiumferrit ($MgFe_2O_4$) sind.

**3.** Verfahren zur Herstellung eines keramischen Körpers nach Anspruch 1, dadurch gekennzeichnet, daß man eine gepulverte Mischung, die aus 20 bis 90 Gew.-% ferromagnetischer Ferritteilchen (A), 80 bis 10 Gew.-% eines anorganischen Materials (B) aus Glas oder kristallisiertem Glas, und gegebenenfalls bis zu 10 Gew.-% der anderen Komponente (C) besteht, sintert.

**4.** Verfahren nach Anspruch 3, dadurch gekennzeichnet, daß die ferromagnetischen Ferritteilchen Magnetit ($Fe_3O_4$),Lithiumferrit ($LiFe_5O_8$) oder Magnesiumferrit ($MgFe_2O_4$) sind.

**5.** Verfahren zur Herstellung eines keramischen Körpers nach Anspruch 1, dadurch gekennzeichnet, daß man eine Schmelze einer Zusammensetzung, die aus 10 bis 60 Gew.-% CaO, 5 bis 50 Gew.-% $SiO_2$, 10 bis 80 Gew.-% $Fe_2O_3$, und gegebenenfalls bis zu 10 Gew.-% der anderen Komponente (C) besteht, abkühlt, um Ferritteilchen (A) während des Kühlens auszufällen, oder um ein Glas auszubilden, und dann gegebenenfalls die Zusammensetzung wiedererhitzt, um Ferritteilchen auszufällen.

## Revendications

**1.** Corps de céramique pour l'utilisation dans le traitement par hyperthermie, lequel corps est constitué de 20 à 90 % en poids de particules de ferrite ferromagnétique (A), 80 à 10 % en poids d'une matière minérale (B), en tant que matrice dans laquelle les particules sont dispersées, et éventuellement jusqu'à 10 % en poids d'un autre composant (C) qui est $Li_2O$, $K_2O$, MgO, SrO, $B_2O_3$, $Al_2O_3$, $TiO_2$, $ZrO_2$, $Nb_2O_5$, $Ta_2O_5$ ou $CaF_2$, la matière minérale (B) étant constituée d'au moins un de la wollastonite, du silicate dicalcique, de l'hydroxyapatite, d'un verre constitué de CaO et de $SiO_2$ et d'un verre cristallisé constitué de CaO et de $SiO_2$, pour que de l'hydroxyapatite soit présente dans une couche superficielle ou soit formée dans une couche superficielle lorsque le corps est inclus dans un tissu vivant.

**2.** Corps de céramique selon la revendication 1, où les particules de ferrite sont faites de magnétite ($Fe_3O_4$), de ferrite de lithium ($LiFe_5O_8$) ou de ferrite de magnésium ($MgFe_2O_4$).

**3.** Procédé de production d'un corps de céramique selon la revendication 1, qui comprend l'étape consistant à fritter une composition mélangée en poudre constituée de 20 à 90 % en poids de particules de ferrite ferromagnétique (A), 80 à 10 % en poids d'une matière minérale (B) faite de verre ou de verre cristallisé et éventuellement jusqu'à 10 % en poids de l'autre composant (C).

**4.** Procédé selon la revendication 3, où les particules de ferrite ferromagnétique sont faites de magnétite ($Fe_3O_4$), de ferrite de lithium ($LiFe_5O_8$) ou de ferrite de magnésium ($MgFe_2O_4$).

**5.** Procédé de production d'un corps de céramique selon la revendication 1, qui comprend l'étape consistant à refroidir une masse fondue d'une composition constituée de 10 à 60 % en poids de CaO, 5 à 50 % en poids de $SiO_2$, 10 à 80 % en poids de $Fe_2O_3$ et éventuellement jusqu'à 10 % en poids de l'autre composant (C), pour précipiter des particules de ferrite (A) lors du refroidissement ou pour former un verre, puis, éventuellement, réchauffer la composition pour précipiter des particules de ferrite.

## FIG.1

## FIG.2

# FIG.3

## FIG.4

Heat Generating Ceramics Body

## FIG.5